# EUROPÄISCHE PATENTSCHRIFT

(11) **EP 1 412 314 B1**
(45) Veröffentlichungstag und Bekanntmachung des Hinweises auf die Patenterteilung: **25.11.2009**
(21) Anmeldenummer: 02762390.9
(22) Anmeldetag: 23.07.2002
(51) Int. Cl.: C07C 47/21, C07C 35/17, A01N 37/02, A01N 35/02

(54) **INSEKTENABWEHRMITTEL**
INSECT REPELLENT
INSECTIFUGE

(30) Priorität: 30.07.2001 DE 10137085
(43) Veröffentlichungstag der Anmeldung: 28.04.2004
(73) Patentinhaber: FULLTEC AG, 6301 Zug (CH)
(72) Erfinder: BENCSITS, Franz, A-3400 Klosterneuburg (AT)
(74) Vertreter: Grünecker, Kinkeldey, Stockmair & Schwanhäusser Anwaltssozietät
(86) Internationale Anmeldenummer: PCT/EP2002/008216
(87) Internationale Veröffentlichungsnummer: WO 2003/011805

(56) Entgegenhaltungen:
- EP-A- 0 216 416
- EP-A- 0 495 684
- WO-A-00/49865
- WO-A-94/04029
- WO-A-96/00056
- WO-A-97/49380
- WO-A-02/055648
- DATABASE WPI Section Ch, Week 199102 Derwent Publications Ltd., London, GB; Class D22, AN 1991-013523 XP002223775 & SU 1 544 435 A (VETERINARY SANIT RE), 23. Februar 1990 (1990-02-23)
- INAGAKI, T. & UEDA, H.: "Enantioselective Esterification of Racemic Terpene Alcohols with Fatty Acids by Pseunomonas sp. NOF-5 Strain" AGRICULTURAL AND BIOLOGICAL CHEMISTRY, Bd. 51, Nr. 5, 1987, Seiten 1345-1348, XP009001982
- DATABASE WPI Section Ch, Week 200223, Derwent Publications Ltd., London, GB; Class C03, AN 2002-172218, XP002223776 & BR 0 001 980 A (RAVENA B B) 02 Januar 2002

## Beschreibung

Die vorliegende Erfindung betrifft ein Insektenabwehrmittel (Repellent) gegen fliegende, stechende, beissende und saugende Insekten sowie Lästlinge der Gattung Acarina (Milben und Zecken).

Repellentien sind chemische Substanzen, die auf Insekten und Acarina abstossend wirken. Große praktische Bedeutung hat ihre Anwendung in der Human- und Veterinärhygiene, wo sie Mensch und Tier vor Befall mit blutsaugenden, stechenden, beissenden und damit nicht nur lästigen, sondern auch potentiell krankheitsübertragenden (Malaria, FSME, Lyme-Borreliose u.v.a.m.) Schädlingen schützen. Bei unmittelbar auf die Haut aufzutragenden Repellentien ist es erforderlich, dass sie hautverträglich, ungiftig, schweiß- und lichtecht und in kosmetischer, damit äusserlich nicht hautbeinträchtigender (Austrocknung, Faltenbildung) und pharmakologisch-gesundheitlicher (Irritationen, Penetration in tiefere Hautschichten und in den Blut- und Lymphkreislauf) Hinsicht einwandfrei sind. Außerdem soll der Schutz der behandelten Hautpartien oder der Schutz von Mensch und Tier durch behandelte Umgebungsgegenstände möglichst lange Zeit anhalten und die Wirkungsbreite der Repellentien möglichst groß sein, d.h. sie sollen gegen möglichst viele, unterschiedliche Schädlinge und Lästlinge wirken.

In der Vergangenheit und in geringerem Maße bis heute wurden und werden etherische Öle, wie Citronell-und Lemongras-, sowie Nelken-, Lavendel- und Eukalyptusöl, sowie Campher als Repellentien eingesetzt, die aber allesamt folgende Nachteile aufweisen:
- bedenkliche Inhaltsstoffe, wie z. B. Eugenol in Nelkenöl, das im Experiment als carzinogen, mutagen und hautreizend befunden wurde, oder Cineol im Öl aus Eucalyptus globulus, das auf der Haut im Stande ist, heftig juckende Exantheme hervorzurufen,
- nur kurzzeitige Wirksamkeit, da die etherischen Öle allesamt schnell durch die Körpertemperatur von der Hautoberfläche abdunsten und damit häufige Nachbehandlungen notwendig sind, um den Schutz zu gewährleisten,
- fehlende Lichtechtheit und damit die permanente Gefahr von Phytosensibilisierung und Produktveränderungen bereits vor Anwendung.

In der jüngeren Vergangenheit wurden sie dadurch überwiegend durch sogenannt synthetische Repellentien ersetzt. Im Stand der Technik angewandte synthetische Repellentien sind z.B. Phtalsäuredimethylester, 1,2-Ethylhexan-1,3-diol, 3,4-Dihydro-2,2-dimethyl-4-oxo-2H-pyran-6-carbonsäure-n-butylester, Bemsteinsäuredipropylester, N,N-Diethyl-3-methyl-benzamid (DEET - auch N,N-Diethyl-m-toluamid) und Pyridin-2,5-dicarbonsäure-di-n-propylester (Ullmanns Encyclopädie der techn. Chemie, 4. Auflage, Bd. 13, S. 237 ff., 1977). In letzter Zeit wird verstärkt Hydroxyethyl-butyl-piperidincarboxylat [1-Piperidincarboxylsäure-2-(2-hydroxyethyl)-1-methylpropylester] eingesetzt. Häufig sind diese synthetischen Repellentien jedoch nicht schweissbeständig, reizen die Schleimhäute und sind ebenso imstande, durch die oberste Hautschicht zu penetrieren und sich damit im Körper zu akkumulieren, wobei die daraus resultierenden Nebenwirkungen noch nicht vollständig erforscht sind, aber berechtigter Verdacht auf schädliche Auswirkungen nahegelegt ist.

Die WO-A-94/04029 beschreibt den Einsatz von Carbonsäuren als Insektenabwehrmittel.

Die WO-A-97/49830 beschreibt verschiedene Insekten abwehrende Stoffe, z.B. auch p-Methan-3,8-diol.

SU-A-1 544 435, EP-A-216 416 und Inagaki, Ueda; Agric. Biol. Chem., 51(5), 1987, 1345-1348 offenbaren den Einsatz von ungesätigten Terpenen in wäßriger Formulierung für verschiedene Zwecke, z.B. zur Abwehr von üblen Gerüchen oder auch Insekten.

Demgemäß liegt der vorliegenden Erfindung die Aufgabe zugrunde, eine Verbindung zur Verfügung zu stellen, die geeignet ist als wirksames Insektenabwehrmittel für den Auftrag direkt auf die Haut, und/oder die Kleidung und/oder andere, den sich schützenwollenden Anwender umgebende Gegenstände und Materialien (Bettwäsche, Tischdecken etc.) auf Basis natürliche und naturidentischer Rohstoffe mit geringstem toxikologischen Risiko, die außerdem über einen möglichst langen Zeitraum hinweg eine hohe Wirksamkeit entfaltet.

Diese Aufgabe wird erfindungsgemäß durch die Zusammensetzung nach Anspruch 1 gelöst. Unter Monoterpenoiden versteht man Dimerisierungsprodukte des Isoprens (2-Methyl-1,3-butadien, C₅H₈), die C₁₀-Grundgerüst haben. Monoterpenoide können acyclisch, mono- und bicyclisch sein.

Bevorzugte Monoterpenoide sind Citronellal oder ein Isomer davon und ein p-Menthenol oder ein Isomer davon. Als p-Menthenol wird bevorzugt Isopulegol oder ein Isomer davon verwendet. Weitere Beispiele für p-Menthenole sind z.B. dargestellt im RÖMPP Lexikon Naturstoffe, 1997, S. 393.

Bevorzugte Ausführungsformen der vorliegenden Erfinding sind in den Ansprüchen 2 bis 6 definiert.

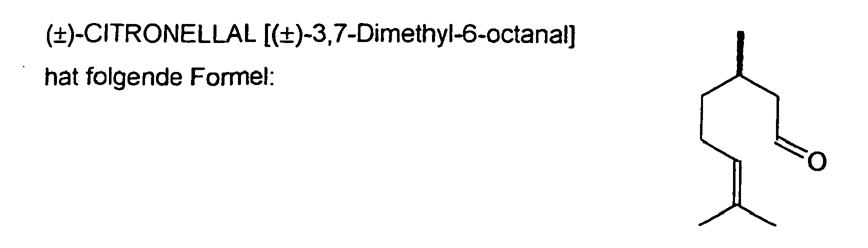

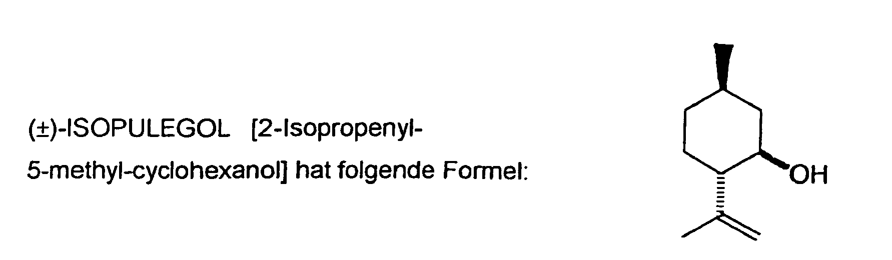

Die ungesättigten Monoterpenoide können als etherisches Öl durch Wasserdampfdestillation aus den Blättern von Eucalyptus citriodora Hook (Kennzeichnung: natürlich; gemäß INCl: Eucalyptus citriodora, CAS: 8000-48-4, EINECS: 283-406-2, Hauptbestandteile: 70 bis 90% Citronellal und Isopulegol) gewonnen werden.

Ebenso möglich ist die Verwendung von synthetisch aus Geraniol, Nerol, Citronellol und/oder Citral hergestelltem Citronellal und dem daraus nach bekannten Methoden der Parfumgrundstoffindustrie isomerisierten Isopulegol.

Die erfindungsgemäß einzusetzende Carbonsäure kann eine natürliche oder synthetische sein, bzw. Mischungen daraus.

Die aus natürlichen Quellen gewonnenen Carbonsäuren können tierischen oder pflanzlichen Ursprungs sein. Bevorzugt werden sie aus fetten oder etherischen Ölen gewonnen, wie Kokosöl, Palmenöl, Kalmusöl, Pelargoniumöl, Quendelöl oder Irisöl, Butter oder Talg. Die zur Gewinnung von Carbonsäuren aus diesen Naturprodukten notwendigen Verfahren sind dem Fachmann bekannt und umfassen Fettspaltung mittels Verseifung oder Hydrolyse im Autoklaven nach dem Twitchell-Verfahren oder die reine Druckspaltung mit Wasserdampf. Als Grundstoffe synthetischen Ursprungs können Alkohole und/oder Aldehyde, sowie aliphatische bzw. acyclische Kohlenwasserstoffe dienen, die nach den bekannten Verfahren, wie Oxo-, Reppe- und Koch-Haaf-Synthese, Carbonylierungsreaktionen oder Verseifung von Nitrilen, zu Fettsäuren umgewandelt werden. Bevorzugte Carbonsäuren, alleine oder in Mischungen, sind Carponsäure, Caprylsäure, Perlargonsäure, Caprinsäure, Undecylensäure (insb. 10-Undecensäure), Undecansäure, Laurinsäure und Tridecansäure. Als Carbonsäure wird bevorzugt Laurinsäure (Carbonsäure C₁₂, Dodecansäure) CH₃(CH₂)₁₀COOH eingesetzt.

Die erfindungsgemäße Zusammensetzung kann als Insektenabwehrmittel verwendet werden.

Weiterhin kann die erfindungsgemäße Verbindung oder die erfindungsgemäße Zusammensetzung in folgenden Fertigprodukten eingesetzt werden: Emulsion, Dispersion, Lotion, Creme, Gel oder Lösung.

Zur Herstellung dieser Fertigprodukte können die allgemein üblichen Präparationsverfahren zum Einsatz kommen. Ebenso können dabei die üblichen Grundstoffe und Additive eingesetzt werden. Sie umfassen die üblicherweise verwendeten Lösungsmittel, Lösungsbeschleuniger, Emulgatoren, Lösungsmittler, Netzmittler, Antischaummittel, Salzbildner, Puffer, Gelbildner, Verdickungsmittel, Filmbildner, Bindemittel, Gleitmittel, Antiklebmittel, Fliessregulierungsmittel, Feuchthalte- und Trockenmittel, Füllstoffe und Hilfsstoffe, wie Antioxidantien, Konservierungsmittel, Geruchskorrigentien und Färbemittel.

Der Begriff Emulsion umfasst alle dispersen Systeme aus zwei oder mehreren miteinander nicht mischbaren Flüssigkeiten, wobei die Emulsionspartner auch als feste Stoffe bei Raumtermperatur vorliegen können. Diese Emulsionen können Makro- oder Mikroemulsionen sein. Typischerweise werde Wasser-in-Öl- oder Öl-in-Wasser-Emulsionen verwendet. Zur Verringerung der Grenzflächenarbeit (aufzuwendende Arbeit zum Emulgieren) werden Emulgatoren verwendet. Emulgatoren sind üblicherweise oberflächenaktive Stoffe, typischerweise mit hydrophilen Endgruppen.

Typische Beispiele davon umfassen:
a) anionische Emulgatoren, d.h. Emulgatoren mit Carboxylat-, Sulfonat-, Sulfat-, Phosphat-, Polyphosphat-, Lactat-, Citrat-, Tartrat-, Glucose- oder Polyglucose-Endgruppen;
b) kationische Emulgatoren, d.h. Emulgatoren mit Aminsalz- oder quartären Ammonium-Endgruppen;
c) amphotere und zwitterionische Emulgatoren, d.h. Emulgatoren mit zwitterionischen Endgruppen oder Betain-Engruppen; sowie
d) nicht-ionische Emulgatoren, d.h. Emulgatoren mit Alkohol-, Polyether-, Glycerin-, Sorbit-, Pentaerythrit-, Saccharose-, Essigsäure und /oder Milchsäure-Resten in der
Endgruppe.

Alle Emulgatoren beinhalten zudem lipophile Endgruppen, wie Alkyl- oder AlkenylReste, jeweils geradkettig, verzweigt oder cyclisch, sowie Aryl- oder Alkylaryl-Reste. Weiterhin können hydrophile Seitengruppen, wie Hydroxyl-, Ester-, Sulfamid-, Amid-, Amin-, Polyamid-, Ether-, Polyether-, Glycerin-, Sorbit-, Pentaerytherit- oder Saccarose-Gruppen, enthalten sein.
Der Begriff Gele umfasst formbeständige, leicht deformierbare, an Flüssigkeiten reiche Systeme aus mindestens zwei Komponenten. Üblicherweise sind diese zwei Komponenten a) eine Flüssigkeit und b) ein fester, kolloidal verteilter Stoff, wie Gelatine, Kieselsäure, Montmorillonit, Bentonit, Polysaccharide, Polyacrylat und Pektine.

Die Hydratisierung erfolgt vorzugsweise direkt bei der Herstellung der wässrig formulierten Fertigprodukte, wobei als Katalysatoren organische Säuren, wie Citronensäure, Benzoesäure, Milchsäure, Sorbinsäure, Apfelsäure, Weinsäure, Gluconsäure, Fumarsäure und Bernsteinsäure, dienen. Prinzipiell sind als Katalysatoren alle Hydroxycarbon- und Dicarbonsäuren einsetzbar, wobei aber aus toxikologischen und dermatologischen Gründen Citronen-, Benzoe-, Milch-, Sorbin-, Apfel- und Weinsäure bevorzugt sind, insofern sie als Katalysator nicht verbraucht werden oder einer chemischen Reaktion unterliegen, sondern im Produkt direkt als natürlich basierende, gut verträgliche pH-Stabilisatoren und Konservierungsmittel dienlich sind.

Unter Hydratisierung ist hier jener Vorgang zu verstehen, der durch chemische Reaktion und kovalent erfolgende Bindung von H und OH aus Wasser an zwei benachbarte Atome erfolgt und mit Hydratation (Solvation in Wasser als Lösungsmittel) nicht verwechselt werden darf.

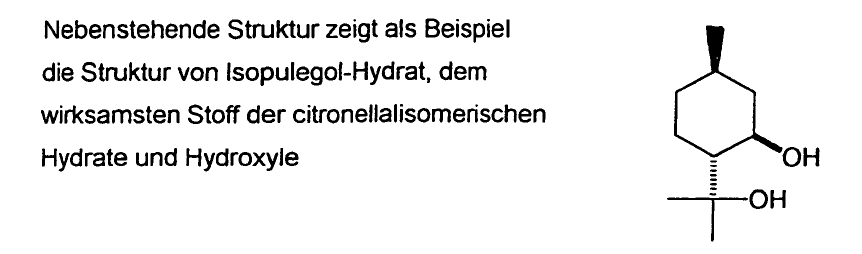

Die Herstellung erfolgt dermassen, dass Wasser auf 38 bis 42°C, vorzugsweise auf 40°C, erwärmt und die Katalysesäure(n) darin aufgelöst wird (werden), die Lösung auf dieser Temperatur gehalten wird, das ungesättigte Monoterpenoid zugefügt und mit einer geigneten Vorrichtung für ca. 6 Stunden gerührt wird, bis die Hydratisierung erfolgt ist, wonach dann im fertigen Produkt als Wirkstoff ein hydratisiertes Monoterpenoid vorliegt, wie mittels MS (Massenspektroskopie) ermittelt werden kann.

Des weiteren kann die Hydratisierung in einer geeigneten Anlage, wie sie in vielen chemischen Fabriken als technischer Standard vorhanden ist, auch mit herkömmlichen Katalysatoren, wie z.B. Metallverbindungen vorgenommen werden und die dabei entstandenen Hydrate dann direkt als Rohstoffe für formulierte Repellents verwendet werden.

Beispiele solcher metallischen Katalysatoren sind: Wolfram (VI)-oxidchlorid, Quecksilber (II)-acetat, Magnesium-methylcarbonat, Mg-trifluormethansulfonat. Wolfram (VI)-oxidchlorid ist ein bevorzugter Katalysator.

Ferner können andere, auf den jeweiligen Reaktor abgestimmte Katalysatoren verwendet werden.

Ein weiterer Vorteil der erfindungsgemäßen Zusammensetzung besteht darin, dass damit stabile alkoholfreie Formulierungen mit nur geringfügiger Emulgatormenge möglich sind, die auf Grund ihres fehlenden Reiz- und Hautaustrocknungspotentials, wie es bei Aufbringung von Alkoholen auf die Haut latent ist, sehr gut für Verwender mit Problemhaut und für Kleinkinder geignet sind.

Die Herstellung erfolgt nach dem gängigen Prinzip der Emulsionsbereitung, indem Wasser auf ca. 65°C erwärmt wird, die Säuren darin gelöst werden und diese wässrige Lösung in die separat bei ca. 55°C geschmolzene C₆-C₁₃ Carbonsäure und einen nicht-ionischen Emulgator, z.B. 'PEG-40 hydrogenated Castor oil', eingebracht, mit einer geeigneten Vorrichtung emulgiert und auf 40°C abgekühlt wird. Bei dieser Temperatur wird Citronellal und/oder Isopulegol eingebracht und so lange gerührt bis über den/die sauren Katalysator(en) die Hydratisierung stattgefunden hat. Einzige Bedingung für den vollständigen Ablauf der Hydratisierung ist die Anwesenheit einer genügend grossen Wassermenge, deren Mindestverhältnis 1 Teil Wasser zu 10 Teilen ungesättigtem Monoterpenoid beträgt.

Ebenso können die Zusammensetzungen mit synthetisch hydratisiertem Monoterpenoid, das wie oben beschrieben produziert wurde, hergestellt werden.

Im folgenden wird die Erfindung anhand von Beispielen erläutert.

### Beispiele

Die folgenden Beispiele 1 bis 12 zeigen als Insektenabwehrmittel geteste Formulierungen. Die Beispiele 1 und 2 enthalten keine hydratisierten Monoterpenoide. In den Beispielen 3 und 4 wurden die Monoterpenoide während der Herstellung des Fertigprodukts hydratisiert und in den Beispielen 5 und 6 erfolgte die Hydratisierung separat synthetisch. Die Beispiele 7 und 8 zeigen erfindungsgemäße Zusammensetzungen, wobei die Hydratisierung wiederum bei der Herstellung des Fertigprodukts erfolgt.

| **Beispiel 1:** | **Beispiel 2:** |
|---|---|
| 50,00 Gew.% Eucalyptus citriodora-öl | 25,00 Gew.% Citronellal |
| 50,00 Gew.% Isopropylalkohol | 25,00 Gew.% Isopulegol |
| | 50,00 Gew.% Isopropylakohol |

| **Beispiel 3:** | **Beispiel 4:** |
|---|---|
| 25,00 Gew.% Wasser | 25,00 Gew.% Wasser |
| 2,00 Gew.% Benzoesäure | 2,00 Gew.% Benzoesäure |
| 0,50 Gew.% Citronensäure | 0,50 Gew.% Citronensäure |
| 25,00 Gew.% Eucalyptus citriodora-Öl | 10,00 Gew.% Citronellal |
| 47,50 Gew.% Isopropylalkohol | 10,00 Gew.% Isopulegol |
| | 52,50 Gew.% Isopropylalkohol |

| **Beispiel 5:** | **Beispiel 6:** |
|---|---|
| 30,00 Gew.% Wasser | 30,00 Gew.% Wasser |
| 10,00 Gew.% Citronellal-hydrat | 10,00 Gew.% Isopulegol-hydrat |
| 60,00 Gew.% Isopropylalkohol | 60,00 Gew.% Isopropylalkohol |

| **Beispiel 7:** | **Beispiel 8:** |
|---|---|
| 66,50 Gew.% Wasser | 66,50 Gew.% Wasser |
| 2,00 Gew.% Benzoesäure | 2,00 Gew.% Benzoesäure |
| 0,50 Gew.% Citronensäure | 0,50 Gew.% Citronensäure |
| 20,00 Gew.% Citronellal | 20,00 Gew.% Isopulegol |
| 1,00 Gew.% PEG-40 Castor oil hydrogenat. | 1,00 Gew.% PEG-40 Castor oil hydrogenat. |
| 10,00 Gew.% Laurinsäure | 10,00 Gew.% Laurinsäure |

| **Beispiel 9:** | **Beispiel 10:** |
|---|---|
| 72,00 Gew.% Wasser | 62,00 Gew.% Wasser |
| 2,00 Gew.% Citronensäure | 2,00 Gew.% Citronensäure |
| 20,00 Gew.% Citronellal | 20,00 Gew.% Isopulegol |
| 1,00 Gew.% PEG-40 Castor oil hydrog. | 1,00 Gew. % PEG-40 Castor oil hydrog. |
| 5,00 Gew.% Laurinsäure | 15,00 Gew.% Laurinsäure |

| **Beispiel 11:** | **Beispiel 12:** |
|---|---|
| 66,50 Gew.% Wasser | 66,50 Gew.% Wasser |
| 2,00 Gew.% Benzoesäure | 2,00 Gew.% Benzoesäure |
| 0,50 Gew.% Citronensäure | 0,50 Gew.% Citronensäure |
| 20,00 Gew.% Citronellal | 20,00 Gew.% Citronellal |
| 1,00 Gew.% PEG-40 Castor oil | 1,00 Gew.% PEG-40 Castor oil |
| hydrogenat. | hydrogenat. |
| 10,00 Gew.% Laurinsäure | 10,00 Gew.% Laurinsäure |

Zu den Beispielen 9 - 12 werden die folgenden Referenzproben hergestellt:

| | **REF 2:** | **REF 3:** | **REF 4:** | **REF 5:** |
|---|---|---|---|---|
| Wasser | 91,50 | 81,50 | 86,50 | 86,50 |
| Benzoesäure | 2,00 | 2,00 | 2,00 | 2,00 |
| Zitronensäure | 0,50 | 0,50 | 0,50 | 0,50 |
| PEG-40 hyd. Castrol Oil | 1,00 | 1,00 | 1,00 | 1,00 |
| Laurinsäure | 5,00 | 15,00 | - | - |
| Caprinsäure | - | - | 10,00 | - |
| Capriylsäure | - | - | - | 10,00 |

Die ungesättigten Monoterpenoide werden als etherisches Öl durch Wasserdampfdestillation aus den Blättern von Eucalyptus citriodora Hook (Kennzeichnung: natürlich; gemäß INCl: Eucalyptus citriodora, CAS: 8000-48-4, EINECS: 283-406-2, Hauptbestandteile: 70 bis 90% Citronellal und Isopulegol) gewonnen.

Aus den später beschriebenen Testergebnissen läßt sich ersehen, dass die Steigerung der Wirksamkeit bei Verwendung von Citronellal oder Isopulegol oder Mischungen der beiden Isomeren (Beispiel 2) gegenüber etherischem Öl aus Eucalyptus citriodora (Beispiel 1) noch nicht signifikant ist.

Ein Anstieg in der Dauer und Intensität der Abhaltewirkung auf alle repräsentativ getesteten Lästlinge tritt ein, wenn hydratisiertes Eucalyptus citriodora-Öl oder Citronellal und/oder Isopulegol verwendet wird (Beipiele 3 bis 6).

Eine überraschende sehr signifikante Wirkungssteigerung hatte die Zugabe einer wie vorstehend definierter Carbonsäure, insbesondere von Laurinsäure C₁₂H₂₄O₂ (Beispiele 7 und 8).

### 1. Testserie Repellens gegen Stechmücken am Menschen

Die Schädlingsabwehrmittel Beispiele 1 bis 8 wurden jeweils an zwei verschiedenen Personen getestet, wobei als Referenz ein, als äußerst wirksam anerkanntes Marktprodukt diente (KIK AKTIV ® - mit 30% DEET (N, N-diethyl-m-toluamide) als Wirkstoff).

Der rechte Unterarm der jeweiligen Versuchsperson wurde auf einer Fläche von ca. 250 cm³ mit dem entsprechenden Testprodukt (BSP 1 bis 8) behandelt. Eine Menge von 2 ml der entsprechenden Testsubstanz wurde auf der Testfläche gleichmäßig verteilt. Die behandelte Unterarmfläche wurde sowohl zum Oberarm als auch zur Handwurzel hin mit einem mückenstichdichten Klebeband über einem kurzen Kunststoffschlauch abgedichtet. Die unbehandelte Hand wurde mit einem dicken Handschuh überzogen und diente so gleichzeitig als Kontrolle für die Stechaktivität der Mücken, da diese bei Stechlust auf diesem absitzen und versuchen, durch das Gewebe hindurch in die darunterliegende Haut zu stechen. Der linke Unterarm wurde ebenso mit Referenzprodukt (REF) behandelt. Als Versuchstiere wurden je Einzelversuch (BSP rechts gegen REF links) etwa 300 bis 400 weibliche Gelbfiebermücken in einem Zuchtkäfig von 40x40x40 cm eingesetzt. Dies ist eine Populationsdichte, wie sie in der freien Natur kaum jemals vorkommt und ermöglicht somit eine gute Differenzierung der Wirksamkeit der einzelnen Substanzen. REF 2 bis REF 5 wurden ebenfalls getestet.

Für den Test wurde die Hand und der präparierte Unterarm, nach 1-stündiger Wartezeit ab Behandlung mit der entsprechenden Testsubstanz, erst der linke Arm mit REF und dann der rechte Arm mit BSP stündlich für jeweils 10 Minuten in den Käfig gehalten und während dieser Zeit die Zahl der Stechmücken notiert, die
(a) durch den Handschuh zu stechen versuchten (positive Kontrolle),
(b) die behandelte Fläche näher als 3 cm anflogen, aber wieder abdrehten (Femhaltewirkung)
(c) auf der behandelten Fläche länger als 2 Sekunden sitzen blieben, aber nicht stachen und
(d) in die behandelte Haut einstachen und Blut saugten.

Jede der zwei Testpersonen testete jedes der acht BSP nacheinander, wobei jeder Proband nur einen Versuch an einem Tag durchführte, um die Gefahr von Produktkumulierungen und eventuellen Kreuzreaktionen der Produkte auf ungenügend gereinigter Haut zu vermeiden.

| **Ergebnisse Testperson 1** | | | | | | | | | |
|---|---|---|---|---|---|---|---|---|---|
| Zeit nach Applikation | Expos.- Dauer | Absitzende auf Handschuh (a) | | Anfliegende Mücken (b) | | Absitzende Mücken (c) | | Anzahl der Stiche (d) | |
| | | **BSP1** | **REF** | **BSP1** | **REF** | **BSP1** | **REF** | **BSP1** | **REF** |
| 1 Std. | 1 min. | 120 | 130 | 32 | 0 | 32 | 0 | 32 | 0 |
| | | BSP2 | REF | BSP2 | REF | BSP2 | REF | BSP2 | REF |
| 1 Std. | 10 min. | 150 | 120 | 48 | 0 | 18 | 0 | 0 | 0 |
| 2 Std. | 1 min. | 100 | 100 | 40 | 0 | 36 | 0 | 32 | 0 |

| | | **BSP3** | **REF** | **BSP3** | **REF** | **BSP3** | **REF** | **BSP3** | **REF** |
|---|---|---|---|---|---|---|---|---|---|
| 1 Std. | 10 min. | 140 | 120 | 0 | 0 | 0 | 0 | 0 | 0 |
| 2 Std. | 10 min. | 100 | 120 | 0 | 0 | 0 | 0 | 0 | 0 |
| 3 Std. | 10 min. | 100 | 100 | 3 | 0 | 0 | 0 | 0 | 0 |
| 4 Std. | 10 min. | 120 | 100 | 8 | 0 | 0 | 0 | 0 | 0 |
| 5 Std. | 10 min. | 150 | 120 | 23 | 6 | 2 | 0 | 0 | 0 |
| 6 Std. | 10 min. | 150 | 130 | 45 | 12 | 8 | 2 | 3 | 0 |

| | | **BSP4** | **REF** | **BSP4** | **REF** | **BSP4** | **REF** | **BSP4** | **REF** |
|---|---|---|---|---|---|---|---|---|---|
| 1 Std. | 10 min. | 100 | 130 | 0 | 0 | 0 | 0 | 0 | 0 |
| 2 Std. | 10 min. | 110 | 110 | 0 | 0 | 0 | 0 | 0 | 0 |
| 3 Std. | 10 min. | 110 | 120 | 0 | 0 | 0 | 0 | 0 | 0 |
| 4 Std. | 10 min. | 130 | 130 | 0 | 0 | 0 | 0 | 0 | 0 |
| 5 Std. | 10 min. | 100 | 150 | 4 | 12 | 0 | 2 | 0 | 0 |
| 6 Std. | 10 min. | 100 | 130 | 9 | 16 | 0 | 6 | 0 | 0 |
| 7 Std. | 10 min. | 150 | 140 | 21 | 33 | 4 | 11 | 0 | 1 |
| 8 Std. | 10 min. | 120 | 100 | 34 | 38 | 17 | 17 | 2 | 6 |

| | | **BSP5** | **REF** | **BSP4** | **REF** | **BSP5** | **REF** | **BSP5** | **REF** |
|---|---|---|---|---|---|---|---|---|---|
| 1 Std. | 10 min. | 100 | 120 | 0 | 0 | 0 | 0 | 0 | 0 |
| 2 Std. | 10 min. | 130 | 100 | 3 | 0 | 0 | 0 | 0 | 0 |
| 3 Std. | 10 min. | 110 | 120 | 8 | 0 | 1 | 0 | 0 | 0 |
| 4 Std. | 10 min. | 100 | 140 | 16 | 0 | 6 | 0 | 2 | 0 |
| 5 Std. | 10 min. | 150 | 160 | 27 | 9 | 11 | 0 | 5 | 0 |

| | | **BSP6** | **REF** | **BSP6** | **REF** | **BSP6** | **REF** | **BSP6** | **REF** |
|---|---|---|---|---|---|---|---|---|---|
| 1 Std. | 10 min. | 120 | 100 | 0 | 0 | 0 | 0 | 0 | 0 |
| 2 Std. | 10 min. | 120 | 120 | 0 | 0 | 0 | 0 | 0 | 0 |
| 3 Std. | 10 min. | 150 | 100 | 0 | 0 | 0 | 0 | 0 | 0 |
| 4 Std. | 10 min. | 120 | 120 | 0 | 0 | 0 | 0 | 0 | 0 |
| 5 Std. | 10 min. | 110 | 120 | 0 | 5 | 0 | 0 | 0 | 0 |
| 6 Std. | 10 min. | 150 | 130 | 4 | 12 | 2 | 5 | 0 | 0 |
| 7 Std. | 10 min. | 130 | 100 | 35 | 20 | 21 | 12 | 14 | 2 |

| 1 | 2 | a | b | c | d | a | b | c | d | a | b | c | d | a | b | c | D |
|---|---|---|---|---|---|---|---|---|---|---|---|---|---|---|---|---|---|
| **Produkt** | | **Beispiel 9** | | | | **Beispiel 10** | | | | **Beispiel 11** | | | | **Beispiel 12** | | | |
| 1 | 10 | 120 | 0 | 0 | 0 | 120 | 0 | 0 | 0 | 110 | 0 | 0 | 0 | 100 | 0 | 0 | 0 |
| 2 | 10 | 110 | 0 | 0 | 0 | 130 | 0 | 0 | 0 | 110 | 0 | 0 | 0 | 90 | 0 | 0 | 0 |
| 3 | 10 | 110 | 0 | 0 | 0 | 120 | 0 | 0 | 0 | 120 | 0 | 0 | 0 | 90 | 0 | 0 | 0 |
| 4 | 10 | 120 | 0 | 0 | 0 | 120 | 0 | 0 | 0 | 110 | 0 | 0 | 0 | 100 | 0 | 0 | 0 |
| 5 | 10 | 120 | 0 | 0 | 0 | 130 | 0 | 0 | 0 | 130 | 0 | 0 | 0 | 110 | 0 | 0 | 0 |
| 6 | 10 | 110 | 2 | 0 | 0 | 120 | 0 | 0 | 0 | 120 | 0 | 0 | 0 | 120 | 0 | 0 | 0 |
| 7 | 10 | 110 | 14 | 4 | 0 | 120 | 0 | 0 | 0 | 120 | 5 | 0 | 0 | 120 | 7 | 0 | 0 |
| 8 | 10 | 50 | 13 | 5 | 3(4)* | 130 | 0 | 0 | 0 | 90 | 10 | 0 | 0 | 100 | 15 | 3 | 0 |
| 9 | | | | | | 120 | 0 | 0 | 0 | 60 | 23 | 8 | 2(8)* | 90 | 5 | 3 | 3(2)* |
| 10 | | | | | | 90 | 5 | 0 | 0 | | | | | | | | |
| 11 | | | | | | 90 | 15 | 2 | 0 | | | | | | | | |
| 12 | | | | | | 70 | 14 | 4 | 1 (9)* | | | | | | | | |

| 1 | 2 | a | b | c | d | a | b | c | d | a | b | c | d | a | b | C |
|---|---|---|---|---|---|---|---|---|---|---|---|---|---|---|---|---|
| **Produkt** | | **REF 2** | | | | **REF 3** | | | | **REF 4** | | | | **REF 5** | | |
| 1 | 10 | 100 | 8 | 2 | 0 | 110 | 0 | 0 | 0 | 130 | 0 | 0 | 0 | 130 | 3 | 0 |
| 2 | 10 | 60 | 30 | 18 | 4(1)* | 130 | 14 | 3 | 0 | 60 | 28 | 12 | 2(5)* | 50 | 10 | 2(6)* |
| 3 | 10 | | | | | 80 | 26 | 12 | 2(2)* | | | | | | | |

| | | | | | | | | | | | | | | | | |
|---|---|---|---|---|---|---|---|---|---|---|---|---|---|---|---|---|
| Legende: 1 = Zeit nach der Applikation des jeweiligen Testproduktes in Stunden 2 = Expositionsdauer innerhalb der 10-minütigen Testdauer je Stunde; bei Stichen Abbruch und Angabe der Minuten der dadurch verkürzten Expositionsdauer a = absitzende Mücken auf dem Handschuh b = anfliegende Mücken, die die behandelte Fläche näher als 3 cm anflogen und wieder abdrehten, ohne abzusitzen und kürzer als 2 Sekunden auf der Testfläche absitzende Mücken c = auf der behandelten Fläche länger als 2 Sekunden absitzende Mücken, die aber nicht stachen und somit, zusammen mit den Mücken des Kriteriums b den Belästigungsfaktor darstellen d = Anzahl der stechenden Mücken vor Abbruch des Versuches * = Abbruch auf Grund von Stichen | | | | | | | | | | | | | | | | |

Die Ergebnisse der BSP1 und BSP2 zeigen, dass nichthydratisiertes Citronellal und Isopulegol, sowohl natürlich in Eucalyptus citriodora-Öl enthalten als auch rein aus der Synthese, nur eine eingeschränkte Repellenswirkung aufweisen. Die direkt bei der Fertigproduktherstellung hydratisierten Produkte BSP3 und BSP4 und auch die extern synthetisch hydratisierten Citronellal und Isopulegol in BSP5 und BSP6 wirken schon besser repellierend, wobei sich zeigt, dass der Hauptwirkstoff Isopulegol-Hydrat ist, da BSP6 eine den Formulierungen BSP3, 4, 5 überlegene Wirksamkeit aufweist, obwohl die Wirksamkeit dieser Produkte (BSP 3,4,5 und BSP 6,7,8} den Bestimmungen zum Erhalt des Gütesiegels der Tropeninstitute weitaus genügen würde. Diese Bestimmungen besagen, dass ein Produkt zuverlässig 4 Stunden vor Stichen schützen muss und nicht mehr als 10% der Gesamtzahl während der Dauer des Testes anfliegenden Mücken auch absitzen dürfen, ohne zu stechen.

Die Tests mit BSP7 und BSP 8 wurden mit einer neuen Referenz durchgeführt, da die Wirkung von KIK AKTIV hinreichend bekannt ist und gezeigt werden sollte, dass die als Synergist eingesetzte Laurinsäure alleine nur eine eingeschränkte Repellenswirkung aufweist, deshalb neu REF1 folgender Zusammensetzung:
76,50 Gew.% Wasser
2,00 Gew.% Benzoesäure
0,50 Gew.% Citronensäure
1,00 Gew.% PEG-40 hydrogenated Castor oil
10,00 Gew.% Laurinsäure

| | | **BSP7** | **REF1** | **BSP7** | **REF1** | **BSP7** | **REF1** | **BSP7** | **REF1** |
|---|---|---|---|---|---|---|---|---|---|
| 1 Std. | 10 min. | 150 | 130 | 0 | 16 | 0 | 4 | 0 | 0 |
| 2 Std. | 10 min. | 120 | 100 | 0 | *23 | 0 | *23 | 0 | *12 |
| 3 Std. | 10 min. | 140 | | 0 | | 0 | | 0 | |
| 4 Std. | 10 min. | 150 | | 0 | | 0 | | 0 | |
| 5 Std. | 10 min. | 130 | | 0 | | 0 | | 0 | |
| 6 Std. | 10 min. | 100 | | 0 | | 0 | | 0 | |
| 7 Std. | 10 min. | 120 | | 8 | | 0 | | 0 | |
| 8 Std. | 10 min. | 130 | | 15 | | 4 | | 0 | |
| 9 Std. | 10 min. | 100 | | 24 | | 10 | | 2 | |

| | | **BSP8** | **REF1** | **BSP8** | **REF1** | **BSP8** | **REF1** | **BSP8** | **REF1** |
|---|---|---|---|---|---|---|---|---|---|
| 1 Std. | 10 min. | 130 | 120 | 0 | 14 | 0 | 6 | 0 | 0 |
| 2 Std. | 10 min. | 120 | 150 | 0 | *33 | 0 | *18 | 0 | *15 |
| 3 Std. | 10 min. | 140 | | 0 | | 0 | | 0 | |
| 4 Std. | 10 min | 130 | | 0 | | 0 | | 0 | |
| 5 Std. | 10 min. | 120 | | 0 | | 0 | | 0 | |
| 6 Std. | 10 min. | 110 | | 0 | | 0 | | 0 | |
| 7 Std. | 10 min. | 120 | | 0 | | 0 | | 0 | |
| 8 Std. | 10 min. | 100 | | 0 | | 0 | | 0 | |
| 9 Std. | 10 min. | 100 | | 5 | | 0 | | 0 | |
| 10 Std. | 10 min. | 100 | | 12 | | 0 | | 0 | |

| | | | | | | | | | |
|---|---|---|---|---|---|---|---|---|---|
| *= Abbruch in der 2. Minute wegen zu vielen Stichen | | | | | | | | | |

Insbesondere die Ergebnisse der Versuche mit BSP8 zeigen die überraschende Verbesserung in Femhaltewirkung und Verlängerung der Wirkungsdauer durch den Synergisten sehr deutlich, aber auch die Ergebnisse aus BSP7 lassen diesen Trend im Vergleich zu BSP4 deutlich hervortreten.

### 2. Testserie Repellens gegen Zecken am Hund

Zur Überprüfung der repellenten Wirkung gegen Zecken als typische Vetreter der Acarina, die auch den für Mensch und Tier gefährlichsten und weitverbreitetsten Repräsentanten dieser Gattung darstellen, wurde ein fünf Tage dauernder Freilandtest durchgeführt.

Von einem Tierschutzhaus wurden sechs Hunde gemischter Rassen und Geschlechter, aber annähernd gleichen Gewichtes mit zwei Hundeführem aus den Reihen des Personals zur Verfügung gestellt, die über die fünf Tage hinweg jeder täglich je 4 Stunden lang je drei Hunde in bekannt zeckenverseuchtem Gebiet (Föhrenwald am Stadtrand Wiener Neustadts) spazieren führten. Täglich gleichbleibend wurden dieselben mit V1 und V2 markierten 2 Hunde mit BSP4 behandelt, wovon jeweils 10 ml mittels Bürste ins Fell eingearbeitet wurden. Die 2 mit V3 und V4 bezeichneten Hunde wurden wie oben beschrieben mit BSP8 behandelt, der Hund REF mit Kik aktiv und der Hund K (Kontrolle) blieb immer unbehandelt. Die beiden Hundeführer wurden täglich vor dem Ausgang am ganzen nackten Körper mit 10 ml BSP8 behandelt, da, obwohl FSME geimpft, die Gefahr einer Übertragung von Borreliose durch etwaige Zeckenstiche vermieden werden sollte und gleichzeitig die vorher bereits im Labor an rasierter Meerschweinschenhaut mit 8 Stunden erhobenen Schutzzeit gegen Zeckenstiche über 4 Stunden am potentiell gefährdeten Menschen bestätigt werden sollte. Als zusatzliche Massnahme zur Risikoverminderung wurden Schuhe, Socken, lange Hosen und die an Armen und Beinen mittels Schnurzug geschlossene Jacke der Hundeführer täglich mit jeweils 5 ml BSP8 volldeckend besprüht. Nach jedem der täglich vierstündigen Spaziergänge wurden die Tiere und auch deren Führer akkuratest auf Zecken untersucht, diese dokumentiert und in beschrifteten Gläsern gesammelt und über 72 Stunden auf eventuelle Mortalität nachbeobachtet.

### Ergebnisse

### 1. Führer 1, Hund K und Hunde V1 und V2

Auf der Haut von Führer 1 wurden über die 5 Tage hinweg keine Zecken aufgefunden, von der Kleidung insgesamt 4 Zecken abgenommen, die sich aber im Gegensatz zu Hund K nicht bis zur Haut vorgearbeitet hatten, sondern schon bei der Abnahme von der Kleidung als immobil erwiesen und sich über die Nachbeobachtungszeit von 72 Stunden nicht mehr erholten, wodurch die bereits im Labortest bemerkte mortale Wirkung von BSP8 auf Zecken bestätigt wurde. Dem unbehandelten Kontrollhund (K) wurden an den 5 Tagen insgesamt 43 Zecken abgenommen, wobei alle Zecken die Nachbeobachtungszeit überlebten, wodurch sichergestellt war, dass im Gebiet Zecken vorhanden und lebenfähig waren.

An V1 und 2 konnten keine Zecken aufgefunden werden.

### 2. Führer 2, Hund REF und Hunde V3 und V4

Auf der Kleidung von Führer 2 waren keine Zecken aufzufinden, ebensowenig auf dessen Haut. Auch V3 und V4 waren zeckenfrei, während auf dem Deckfell von REF an den 5 Tagen insgesamt 9 Zecken aufgefunden wurden, die die Nachbeobachtungszeit von 72 Stunden auch problemlos überlebten. Sie wurden dann den Gläsern entnommen und einem Meerschwein im Labor auf die rasierte Flanke angesetzt, wo sie sofort eine geeignete Einstichstelle suchten und sich nach Erreichung des Haarkleides einzubohrten, worauf sie mit einer Pinzette abgenommen und nach den Vorschriften des Immersionstests des Ektoparasitenscreenings 5 Minuten lang in REF getaucht, nach 72 Stunden weiterer Beobachtungszeit wieder dem Meerschwein angesetzt wurden und wieder begannen sich einzubohren.

Als Ergebnis zeigte sich, dass REF zwar eine akzeptable Repellens auf Zecken, aber keine mortale Wirkung aufweist. Die 43, dem unbehandelten Hund K abgenommenen Zecken wurden 5 Minuten lang in BSP8 getaucht und waren binnen 30 Minuten tot.

### Ergebnis Labortest am Meerschweinchen

Zwei adulten weiblichen Meerschweinchen wurden die rechten Flanken im Ausmaß ca. 4x4 cm rasiert und die Haut und die umgebenden behaarten Stellen per Hand mit ca. 2 ml BSP8 vollflächig deckend behandelt. Danach wurde jede Stunde jedem Meerschweinchen je 1 laborgezogene, adulte, zwei Wochen lang ausgehungerte Zecke mittels Pinzette in die Mitte der behandelten, rasierten Fläche aufgesetzt und deren Verhalten beobachtet. Die Meerschweinchen waren dazu nicht wie üblich sediert, um eine möglichst naturgetreue Situation zu simulieren, sondern wurden mit Salat gefüttert und blieben ziemlich ruhig, da sie an menschliche Umgebung gewöhnt sind.

Auffallend war sofort, dass die Zecken nicht, wie sonst auf unbehandelten Meerschweinchen vielfach beobachtet, wandernd begannen, eine für einen Einstich günstige Stelle zu suchen (normalerweise eine Beugefalte unter den Hinterbeinen), sondern sich über einige Minuten auf der behandelten Fläche in einem engen Kreis bewegten und dann bei der geringsten Bewegung ihrer Wirte auf den Boden des Käfigs abfielen, obwohl Zecken normalerweise mit ihren Haltekrallen selbst auf glatten Flächen Halt zu finden vermögen. Die abgefallenen Zecken wurden sofort in beschriftete Gläser verbracht und über 72 Stunden beobachtet, wonach alle tot waren.

Keine der insgesamt 16 Zecken, die innerhalb der 8 Stunden den Meerschweinen angesetzt wurden, bohrte sich ein und begann Blut zu saugen, sondern alle starben überraschenderweise infolge Produktkontakt mit BSP8.

## Patentansprüche

1. Zusammensetzung, umfassend mindestens eine hydratisierte Verbindung eines ungesättigten Monoterpenoids zusammen mit mindestens einer C₆-C₁₃-Carbonsäure.

2. Zusammensetzung nach Anspruch 1, wobei das Monoterpenoid Citronellal oder ein Isomer davon ist.

3. Zusammensetzung nach Anspruch 1, wobei das Monoterpenoid ein p-Menthenol oder ein Isomer davon ist :

4. Zusammensetzung nach Anspruch 3, wobei das p-Menthenol Isopulegol oder ein Isomer davon ist.

5. Zusammensetzung nach einem der Ansprüche 1 bis 4, wobei die Carbonsäure Laurinsäure ist.

6. Verwendung einer Zusammensetzung nach einem der Anspruche 1 bis 5 zur Herstellung eines Insektenabwehrmittel.

## Claims

1. Composition comprising at least one hydrated compound of an unsaturated monoterpenoid together with at least one C₆-C₁₃ carboxylic acid.

2. Composition according to claim 1, wherein the monoterpenoid is citronellal or an isomer thereof.

3. Composition according to claim 1, wherein the monoterpenoid is a p-menthenol or an isomer thereof.

4. Composition according to claim 3, wherein the p-menthenol is isopulegol or an isomer thereof.

5. Composition according to any one of claims 1 to 4, wherein the carboxylic acid is lauric acid.

6. Use of a composition according to any one of claims 1 to 5 for making an insect repellent.

## Revendications

1. Composition comprenant au moins un composé hydraté d'un monoterpénoïde insaturé conjointement avec au moins un acide carboxylique en C₆ - C₁₃.

2. Composition selon la revendication 1, dans laquelle le monoterpénoïde est le citronellal ou un isomère de celui-ci.

3. Composition selon la revendication 1, dans laquelle le monoterpénoïde est un p-menthènol ou un isomère de celui-ci.

4. Composition selon la revendication 3, dans laquelle le p-menthénol est l'isopulégol ou un isomère de celui-ci.

5. Composition selon l'une des revendications 1 à 4, dans laquelle l'acide carboxylique est l'acide laurique.

6. Utilisation d'une composition selon l'une des revendications 1 à 5, pour la préparation d'un agent répulsif des insectes.
